# EUROPÄISCHE PATENTSCHRIFT

(11) **EP 1 850 883 B1**
(45) Veröffentlichungstag und Bekanntmachung des Hinweises auf die Patenterteilung: **27.03.2013**
(21) Anmeldenummer: 06706292.7
(22) Anmeldetag: 19.01.2006
(51) Int. Cl.: A61L 2/20, A61L 2/22, B65B 55/02, B67B 1/03

(54) **VORRICHTUNG ZUM ZUFÜHREN VON IN SÄCKEN ANGELIEFERTEN STERILEN VERSCHLÜSSEN IN EINE ABFÜLLANLAGE FÜR FLASCHEN ODER DGL.**
DEVICE FOR FEEDING STERILE CLOSURES, WHICH ARE DELIVERED IN BAGS, INTO A FILLING SYSTEM FOR BOTTLES OR THE LIKE
DISPOSITIF POUR ACHEMINER DES SYSTEMES DE BOUCHAGE STERILES FOURNIS DANS DES SACS, DANS UNE INSTALLATION DE REMPLISSAGE DE BOUTEILLES OU SIMILAIRES

(30) Priorität: 22.02.2005 DE 202005002903 U
(43) Veröffentlichungstag der Anmeldung: 07.11.2007
(73) Patentinhaber: KRONES Aktiengesellschaft, 93073 Neutraubling (DE)
(72) Erfinder: FISCHER, Sven, 93083 Obertraubling (DE); MARTINI, Oliver, 3600 Thun (CH)
(86) Internationale Anmeldenummer: PCT/EP2006/000434
(87) Internationale Veröffentlichungsnummer: WO 2006/089603

(56) Entgegenhaltungen:
- DE-A1- 1 460 621
- DE-A1- 3 423 436

## Beschreibung

Die Erfindung betrifft eine Vorrichtung zum Zuführen von sterilen Verschlüssen gemäß dem Oberbegriff des Anspruchs 1.

Es ist bereits eine derartige Vorrichtung in Gebrauch, bei der die Fördereinrichtung als Mattenkette ausgebildet ist, auf der die Säcke aufliegen.

Mit dieser bekannten Vorrichtung ist keine vollständige Sterilisation der Säcke möglich, da deren Außenfläche teilweise durch die Mattenkette abgedeckt ist. Es ist daher nicht auszuschließen, dass nach dem Öffnen der Säcke innerhalb des Gehäuses die austretenden Verschlüsse durch mit den Säcken eingeschleppte Keime wieder infiziert werden.

Der Erfindung liegt die Aufgabe zugrunde, bei einer Vorrichtung der Eingangs genannten Art mit einfachen Mitteln eine Reinfektion der Verschlüsse nach dem Öffnen der Säcke zuverlässig zu verhindern.

Diese Aufgabe wird erfindungsgemäß mit der Fördereinrichtung gemäß Patentanspruch 1 gelöst.

Auf diese Weise ist die Außenfläche der Säcke praktisch vollständig zugänglich und kann innerhalb der Sterilisationseinrichtung vollständig desinfiziert werden. Eine Reininfektion der Verschlüsse vor dem Passieren der Austrittsöffnung in die Abfüllanlage ist daher ausgeschlossen.

Besonders vorteilhaft bei der Erfindung ist der magnetische Antrieb des Hängeförderers durch außerhalb des Gehäuses angebrachte Antriebsmittel. Hierdurch wird eine besonders einfache Abdichtung des Gehäuses möglich und die im Gehäuse umlaufenden Bereiche des Hängeförderers bleiben immer steril.

Vorteilhafte Weiterbildungen der Erfindung sind in den Unteransprüchen enthalten.

Im nachstehenden wird ein Ausführungsbeispiel der Erfindung anhand der Zeichnungen beschrieben.

Es zeigen:
- Fig. 1: die schematische Draufsicht auf eine Vorrichtung zum Zuführen von Verschlüssen
- Fig. 2: die Seitenansicht der Vorrichtung nach Figur 1
- Fig. 3: die vergrößerte Seitenansicht eines Teilbereichs des Hängeförderers
- Fig. 4: die vergrößerte Draufsicht auf das Gehäuse der Vorrichtung nach Figur 1 bis 3
- Fig. 5: die schematische Seitenansicht einer Vorrichtung mit zwei Hängeförderern und zwei Sterilisationskammern.

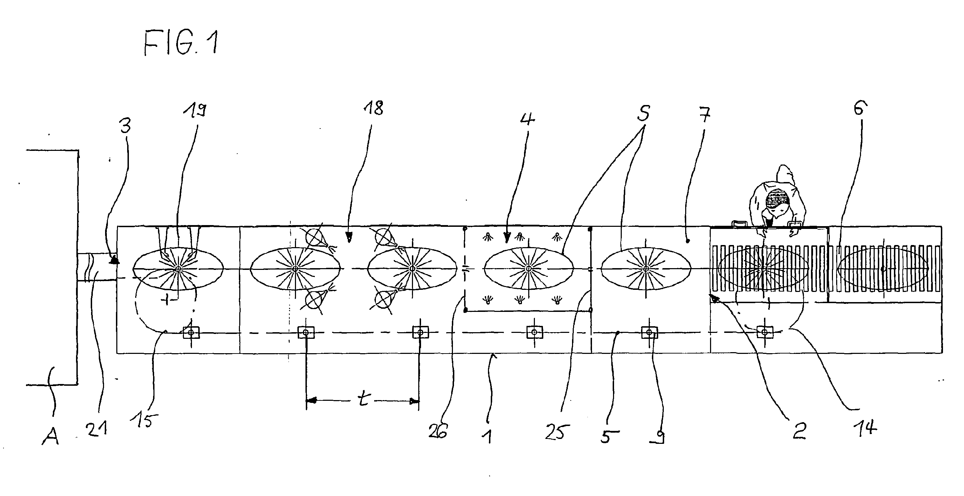

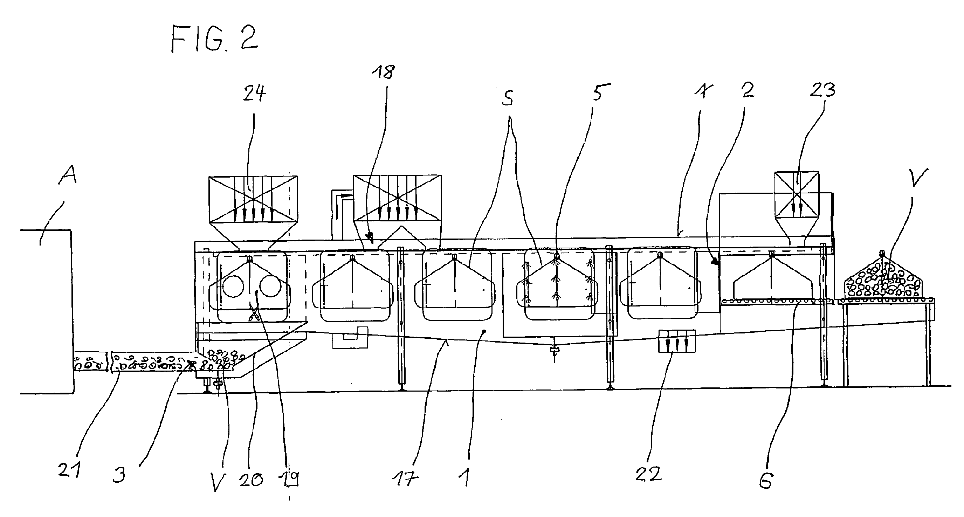

Die Vorrichtung nach den Figuren 1 bis 4 ist zum Einführen von in Säcken S angelieferten Verschlüssen V in eine Abfüllanlage A zum sterilen Befüllen von Flaschen mit einem Erfrischungsgetränk eingerichtet. Vorzugsweise handelt es sich um wiederverschließbare Trinkverschlüsse (Sportcap). Derartige Trinkverschlüsse enthalten schwer zugängliche Hohlräume, die bei einer normale Sterilisation in einem Tauchbad oder dgl. nicht ausreichend desinfiziert werden können. Es ist daher üblich, derartige Trinkverschlüsse in flexiblen Säcken aus Kunststofffolie gasdicht abzupacken und dann mittels Gammastrahlung zu sterilisieren. Auch die Verwendung von Doppelsäcken, wovon der Äußere die Hauptverschmutzung während des Transports aufnimmt, ist üblich.

Die Vorrichtung weist ein längliches Gehäuse 1 mit im Wesentlichen rechteckigen Querschnitt auf, an dessen rechter Stirnseite eine Eintrittsöffnung 2 für die verschlossenen Säcke S und an dessen gegenüberliegender Stirnseite eine Austrittsöffnung 3 für die aus den Säcken S entnommenen sterilen Verschlüsse V ausgebildet ist. An der Eintrittsöffnung 2 ist eine kurze Rollbahn 6 vorgesehen, die teilweise innerhalb und teilweise außerhalb des Gehäuses 2 liegt. Hierdurch wird das manuelle Einführen der Säcke S in das. Gehäuse 1 erleichtert.

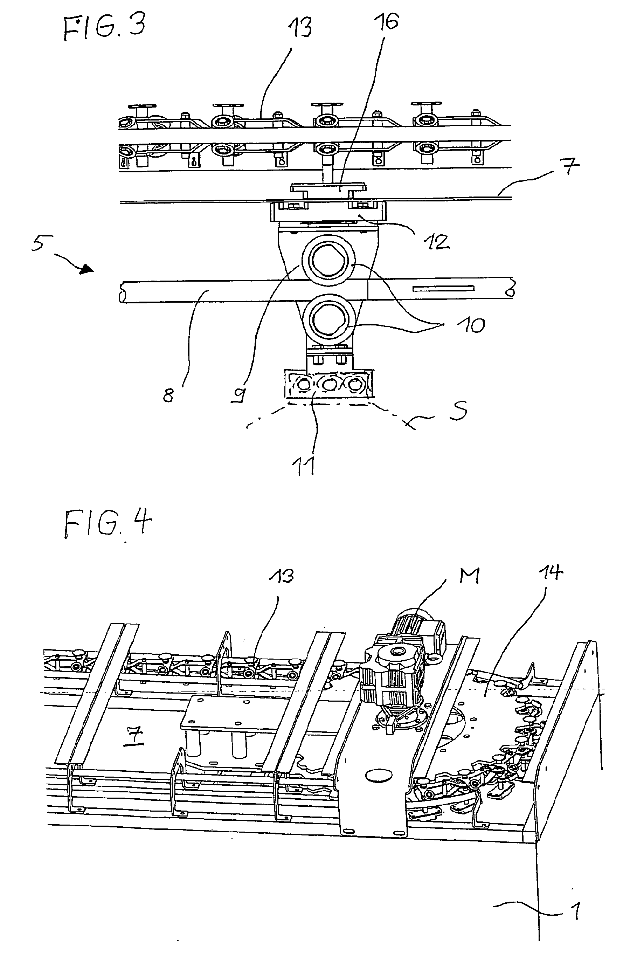

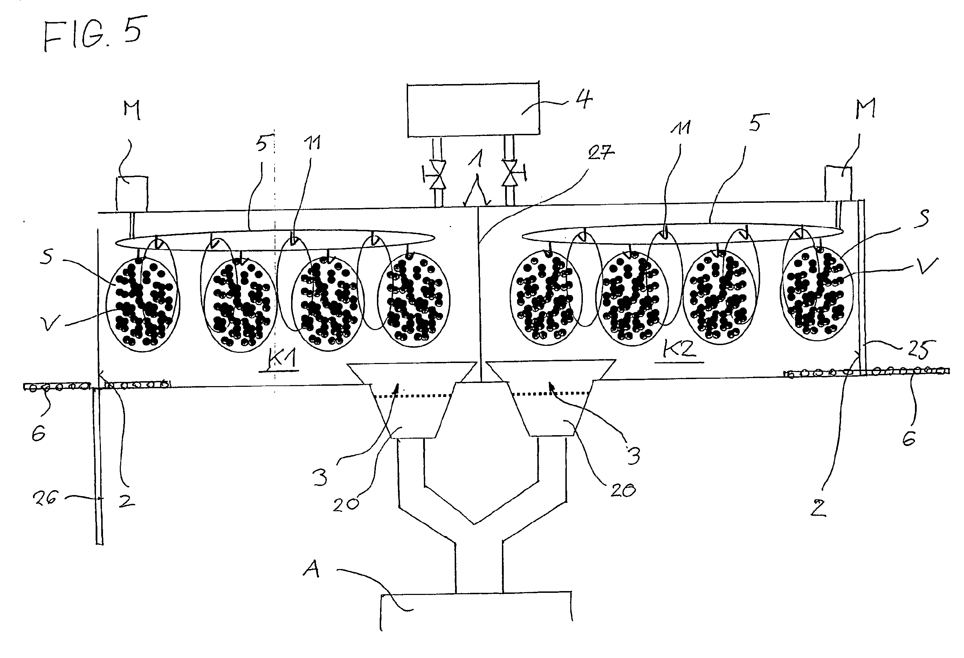

An der Oberseite des Gehäuses 1, genauer gesagt an dessen horizontaler Deckplatte 7 ist ein Hängeförderer 5 für die Säcke S angebracht. Der Hängeförderer 5 weist eine an der Innen- bzw. Unterseite der Deckplatte 7 befestigte ovale Führungsschiene 8 auf, an der mehrere einzelne Läufer 9 mittels Rollen 10 verschiebbar geführt sind. Jeder Läufer 9 besitzt an seiner Unterseite eine Aufnahme 11 für den hängenden Transport jeweils eines Sacks S, beispielsweise einen Haken (Fig. 5) oder mehrere horizontale Bolzen (Fig. 3) auf. An der Oberseite jedes Läufers 9 ist ein Dauermagnet 12 befestigt, der mit geringem Abstand unterhalb der Deckplatte 7 liegt.

An der Ober- bzw. Außenseite der Deckplatte 7 ist eine die Führungsschiene 8 überdeckende endlose Gliederkette 13 mit zwei Umlenkrädern 14, 15 angeordnet, die gleichfalls mit Dauermagneten 16 versehen ist. Diese liegen dicht über der Deckplatte 7 und korrespondieren mit den Dauermagneten 12 der Läufer 9. Bei einem Umlauf der Gliederkette 13 in Pfeilrichtung gemäß Fig. 1 werden somit die Läufer 9 durch Magnetkraft mitgenommen. Der Antrieb der Gliederkette 13 erfolgt durch einen Motor M, der auf eines der Umlenkräder 15 einwirkt.

Der Motor M wird vorzugsweise intermittierend betrieben, entweder automatisch oder manuell gesteuert, wobei die Gliederkette 13 bei jedem Arbeitstakt ein Ein- oder Mehrfaches des Abstands t zwischen zwei Läufern 9 zurücklegt. Der Abstand t ist etwas größer als die maximale Breite eines Sacks S.

Die Gliederkette 13 kann ähnlich wie die Läufer 9 mittels nicht gezeigter Rollen auf einer nicht gezeigten Führungsschiene geführt sein. Sie kann auch mehrere Dauermagneten 16 aufweisen, als Läufer 9 vorhanden sind, wodurch der Abstand zwischen den Läufern 9 variiert werden kann.

Entlang dem in Figur 1 von rechts nach links, d. h. von der Eintrittsöffnung 2 zur Austrittsöffnung 3 gerichteten Umlaufbereich der Läufer 9 sind die eigentlichen Arbeitstationen der Vorrichtung vorgesehen. Als erstes kommt mit einem Abstand von mindestens einer Sackbreite zur Eintrittsöffnung 2 die Sterilisationseinrichtung 4. In dieser wird der jeweilige Sack S mittels mehrerer Düsen von allen Seiten mit einer wässrigen Peressigsäurelösung intensiv besprüht. Unterhalb der Sterilisationseinrichtung 4 ist eine Auffangwanne 17 für die Sterilisationslösung vorgesehen, die sich vorzugsweise über die gesamte Länge des Gehäuses 1 erstreckt.

Als nächstes kommt eine Trocknungseinrichtung 18 in der die jeweiligen Säcke S aus mehreren Düsen allseitig mit warmer Sterilluft intensiv beaufschlagt werden.

Am Ende des Gehäuses 1 schließlich folgt die Entnahmeeinrichtung 19, die zwei elastische Handschuheingriffe für die Bedienungsperson umfasst, so dass diese mit einem geeigneten Werkzeug die Säcke S am unteren Ende aufschneiden kann. Weiter umfasst die Entnahmeeinrichtung 19 einen Trichter 20 für die aus den Säcken S herausfallenden Verschlüsse V, der diese auf einen Vibrationsförderer 21 oder dgl. aufgibt. Dieser ist allseitig geschlossen und führt die Verschlüsse zu der eingehausten Abfüllanlage A.

Im Bereich zwischen der Eintrittsöffnung 2 und der Sterilisationseinrichtung 4 ist eine Absaugeinrichtung 22 angeordnet, um ein Austreten von Sterilisationsmitteldämpfen zu verhindern. Ferner ist im Bereich der Eintrittsöffnung 2 und im Bereich der Austrittsöffnüng 3 jeweils eine Einrichtung 23, 24 zum Einblasen von Sterilluft vorgesehen, um ein Eintreten von verkeimter Umgebungsluft in das Gehäuse zuverlässig zu verhindern.

Zur besseren Abdichtung der Sterilisationseinrichtung 4 kann diese im Einlauf und/oder im Auslauf mit jeweils einer Schleuse 25, 26 versehen sein. Jede Schleuse besteht im einfachsten Falle aus ein oder zwei Klapptüren, die durch den Sack S geöffnet und danach mittels Federkraft geschlossen werden.

Die geöffneten und entleerten Säcke S laufen entlang dem in Figur 1 von links nach rechts, d. h. von der Austrittsöffnung 3 zur Eintrittsöffnung 2 gerichteten Umlaufbereich zurück zur Eintrittsöffnung 2 und werden dort durch die Bedienungsperson von den Aufnahmen 11 herabgenommen und durch einen vollen Sack S ersetzt.

Werden die Verschlüsse V in sogenannten Doppelsäcken S angeliefert, so wird der äußere Sack kurz vor dem Aufhängen der inneren Säcke S an den Aufnahmen 11 entfernt. Auf diese Weise wird verhindert, dass grobe, vom Transport her rührende Verschmutzungen in die Vorrichtung gelangen, so dass nur der ohnehin sehr reine bzw. keimarme Innensack sterilisiert werden muss.

Bei der Vorrichtung nach Figur 5 ist das Gehäuse 1 durch eine starre Trennwand 27 in zwei in etwa gleich große Kammern K1, K2 unterteilt. Jede Kammer weist eine eigene Eintrittsöffnung 2, die durch eine Schleuse 25, 26 verschließbar ist, und nahe der Trennwand 27 eine eigene Austrittsöffnung 3 mit einem Trichter 20 auf. Außerdem ist an jeder Eintrittsöffnung eine Rollbahn 6 vorgesehen. An der Oberseite jeder Kammer ist ein eigener Hängeförderer 5 vorgesehen, der im Aufbau dem Hängeförderer nach Figur 3 und 4 entspricht. Außerdem sind beide Kammern abwechselnd mit einer Sterilisationseinrichtung 4 zu verbinden, die beispielsweise H₂O₂-Dampf in die Kammer einbläst.

Mit der vorstehend beschriebenen Vorrichtung nach Fig. 5 ist ein chargenweiser Betrieb möglich. Hierbei werden die beiden Kammern K1 und K2 abwechselnd entweder mit Sterilisationsmittel beaufschlagt, wobei die jeweilige Schleuse 25, 26 geschlossen ist, oder bei geöffneter Schleuse 25, 26 mit vollen Säcken S bestückt und von leeren Säcken S befreit. Zum Entleeren werden dann nach einer ausreichenden Sterilisationszeit bei taktweisem Betrieb des Hängeförderers 5 die Säcke S nacheinander über den jeweiligen Trichter 20 gefahren, aufgeschnitten und entleert.

Die vorstehend beschriebenen Vorrichtungen ermöglichen auf Grund der allseitigen zugänglichkeit des Säcke S eine intensive Behandlung und Sterilisation so dass die Zufuhr von verkeimten Verschlüssen V in die Abfüllanlage A zuverlässig verhindert wird. Gleichzeitig wird in der Sterilisationseinrichtung der innerhalb des Gehäuses 1 befindliche Bereich des Hängeförderers 5 dauerhaft sterilisiert, während der außenliegende Antriebsbereich des Hängeförderers 5 auf Grund der magnetischen Kraftübertragung in der normalen Umgebung angeordnet sein kann und daher nicht sterilisiert werden muss.

## Patentansprüche

1. Vorrichtung zum Zuführen von in Säcken (S) angelieferten sterilen Verschlüssen (V) in eine Abfüllanlage (A) für Flaschen oder dgl., mit einem Gehäuse (1), das eine Eintrittsöffnung (2) für die Säcke, eine Austrittsöffnung (3) für die Verschlüsse und eine Sterilisationseinrichtung (4) aufweist, wobei im Gehäuse eine die Säcke transportierende Fördereinrichtung (5) vorgesehen ist, **dadurch gekennzeichnet, dass** die Fördereinrichtung für die Säcke (S) als Hängeförderer (5) ausgebildet ist und eine innerhalb des Gehäuses (1) angeordnete endlose Führung (8) sowie an dieser beweglich gelagerte Läufer (9) aufweist, wobei die Läufer (9) mit Aufnahmen (11) zum hängenden Transport der Säcke (S) ausgestattet sind und die Läufer (9) mit Magneten (12) versehen sind, die mit außerhalb des Gehäuses (1) angeordneten, antreibbaren Magneten (16) zusammenwirken.

2. Vorrichtung nach Anspruch 1, **dadurch gekennzeichnet, dass** der Hängeförderer (5) nacheinander die Sterilisationseinrichtung (4) für die Säcke (S), eine Trocknungseinrichtung (18) für die Säcke (S) und eine Entnahmeeinrichtung (19) für die Verschlüsse (V) durchläuft.

3. Vorrichtung nach Anspruch 1 oder 2, **dadurch gekennzeichnet, dass** die außerhalb des Gehäuses (1) angeordneten Magneten (16) an einer endlosen Gliederkette (13) angeordnet sind, die über zwei Umlenkräder (14, 15) umläuft, wovon eines durch einen Motor (M) antreibbar ist.

4. Vorrichtung nach einem der Ansprüche 1 bis 3, **dadurch gekennzeichnet, dass** der Hängeförderer (5) im Bereich der Eintrittsöffnung (2) mit einer Rollbahn (6) für die Säcke (S) und im Bereich der Austrittsöffnung (3) mit einem Trichter (20) für die Verschlüsse (V) überlappt.

5. Vorrichtung nach einem der Ansprüche 1 bis 4, **dadurch gekennzeichnet, dass** zwei getrennte Sterilisierkammern (K1, K2) vorgesehen sind, von denen jede einen eigenen Hängeförderer (5), eine eigene Eintrittsöffnung (2) und eine eigene Austrittsöffnung (3) aufweist, wobei die beiden Kammern abwechselnd an eine Sterilisationseinrichtung (4) anschließbar sind.

6. Vorrichtung nach Anspruch 5, **dadurch gekennzeichnet, dass** die beiden Kammern (k1, K2) in einem gemeinsamen Gehäuse (1) unter Zwischenschaltung einer Trennwand (27) angeordnet sind.

7. Vorrichtung nach Anspruch 5 oder 6, **dadurch gekennzeichnet, dass** die Eintrittsöffnung (2) jeder Kammer (K1, K2) durch eine eigene Schleuse (25, 26) verschließbar ist.

## Claims

1. Device for feeding sterile closures (V) which are delivered in bags (S) into a filling system (A) for bottles or the like, comprising a housing (1) which has an entrance opening (2) for the bags, an exit opening (3) for the closures and a sterilisation device (4), a conveyor device (5) which transports the bags being provided in the housing, **characterised in that** the conveyor device for the bags (S) is in the form of a suspension conveyor (5) and comprises an endless guide (8) arranged inside the housing (1) and runners (9) mounted movably thereon, the runners (9) being equipped with receiving devices (11) for the suspended transportation of the bags (S) and the runners (9) being provided with magnets (12) which cooperate with driveable magnets (16) arranged outside the housing (1).

2. Device according to Claim 1, **characterised in that** the suspension conveyor (5) passes successively through the sterilisation device (4) for the bags (S), a drying device (18) for the bags (S) and a removal device (19) for the closures (V).

3. Device according to Claim 1 or 2, **characterised in that** the magnets (16) arranged outside the housing (1) are arranged on an endless link chain (13) which circulates via two deflection wheels (14, 15), one of which is driveable by a motor (M).

4. Device according to any one of Claims 1 to 3, **characterised in that** the suspension conveyor (5) overlaps a roller track (6) for the bags (S) in the region of the entrance opening (2) and overlaps a funnel (20) for the closures (V) in the region of the exit opening (3).

5. Device according to any one of Claims 1 to 4, **characterised in that** two separate sterilisation chambers (K1, K2) are provided, each of which has its own suspension conveyor (5), its own entrance opening (2) and its own exit opening (3), the two chambers being connectable alternately to a sterilisation device (4).

6. Device according to Claim 5, **characterised in that** the two chambers (K1, K2) are arranged in a common housing (1) with a dividing wall (27) located between them.

7. Device according to Claim 5 or 6, **characterised in that** the entrance opening (2) of each chamber (K1, K2) is closable by means of its own drop gate (25, 26).

## Revendications

1. Dispositif de transfert d'éléments de fermeture stériles (V) fournis dans des sacs (S) dans une installation de remplissage (A) de bouteilles ou similaire comportant un boîtier (1) comprenant une ouverture d'entrée (2) des sacs, une ouverture de sortie (3) des éléments de fermeture et un dispositif de stérilisation (4), un dispositif de transport des sacs (S) étant monté dans le boitier,
**caractérisé en ce que**
le dispositif de transport des sacs (S) est réalisé sous la forme d'un transporteur par suspension (5), et comporte un élément de guidage sans fin (8) monté à l'intérieur du boitier (1) ainsi qu'un tapis rotatif (9) logé mobile sur celui-ci, ce tapis de transport (9) étant équipé de logements (11) pour le transport suspendu des sacs (S) et d'aimants (12) qui coopèrent avec des aimants actionnables (16) montés à l'extérieur du boitier (1).

2. Dispositif conforme à la revendication 1,
**caractérisé en ce que**
le transporteur par suspension (5) traverse successivement le dispositif de stérilisation (4) des sacs (S), un dispositif de séchage (18) des sacs (S) et un dispositif (19) de prélèvement des éléments de fermeture (V).

3. Dispositif conforme à la revendication 1 ou 2,
**caractérisé en ce que**
les aimants (16) situés à l'extérieur du boitier (1) sont montés sur une chaine à maillons sans fin (13) qui tourne autour de deux roues de renvoi (14, 15) dont l'une peut être entrainée par un moteur (M).

4. Dispositif conforme à l'une des revendications 1 à 3,
**caractérisé en ce que**
le transporteur par suspension (5) chevauche une piste de roulement (6) pour les sacs (S) dans la zone de l'ouverture d'entrée (2) et une trémie (20) pour les éléments de fermeture (V) dans la zone de l'ouverture de sortie (3).

5. Dispositif conforme à l'une des revendications 1 à 4,
**caractérisé en ce qu'**
il comporte deux chambres de stérilisation séparées (K1, K2) équipées chacune d'un transporteur par suspension (5) propre, d'une ouverture d'entrée (2) propre et d'une ouverture de sortie (3) propre, ces deux chambres pouvant être alternativement raccordées à un dispositif de stérilisation (4).

6. Dispositif conforme à la revendication 5,
**caractérisé en ce que**
les deux chambres (K1, K2) sont montées dans un boitier (1) commun avec interposition d'une paroi de séparation (27).

7. Dispositif conforme à la revendication 5 ou 6,
**caractérisé en ce que**
l'ouverture d'entrée (2) de chacune des chambres (K1, K2) peut être fermée par une écluse (25, 26).
